(19) Europäisches Patentamt
European Patent Office
Office européen des brevets

(11) EP 2 802 335 B1

(12) # FASCICULE DE BREVET EUROPEEN

(45) Date de publication et mention de la délivrance du brevet:
**03.03.2021 Bulletin 2021/09**

(21) Numéro de dépôt: **13700382.8**

(22) Date de dépôt: **14.01.2013**

(51) Int Cl.:
***A61K 36/54*** (2006.01) ***C11B 1/04*** (2006.01)
***C11B 1/06*** (2006.01) ***C11B 1/10*** (2006.01)
***C11B 3/12*** (2006.01) ***A61P 19/02*** (2006.01)
***A61P 19/04*** (2006.01)

(86) Numéro de dépôt international:
**PCT/EP2013/050583**

(87) Numéro de publication internationale:
**WO 2013/104795 (18.07.2013 Gazette 2013/29)**

(54) **UTILISATION DE NOYAU D'AVOCAT POUR OBTENIR UNE HUILE D'AVOCAT ENRICHIE EN ALKYLS POLYOLS ET/OU EN LEURS DÉRIVÉS ACÉTYLÉS**

VERWENDUNG VON AVOCADO KERNEN ZUR HERSTELLUNG VON AVOCADOÖL ANGEREICHERT AN ALKYL POLYOLEN UND/ODER DEREN AZETYLIERTEN DERIVATEN

USE OF AVOCADO KERNEL TO OBTAIN AVOCADO OIL ENRICHED IN ALKYL POLYOLS AND/OR THEIR ACETYLATED DERIVATIVES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.01.2012 FR 1250368**

(43) Date de publication de la demande:
**19.11.2014 Bulletin 2014/47**

(73) Titulaire: **Laboratoires Expanscience**
**92048 Paris La Défense Cedex (FR)**

(72) Inventeurs:
- **MSIKA, Philippe**
  **F-78000 Versailles (FR)**
- **LEGRAND, Jacques**
  **F-61290 Neuilly Sur Eure (FR)**
- **GARNIER, Sébastien**
  **06650 Le Rouret (FR)**

(74) Mandataire: **Regimbeau**
**20, rue de Chazelles**
**75847 Paris Cedex 17 (FR)**

(56) Documents cités:
**WO-A1-2013/098293** **WO-A2-2004/012496**
**WO-A2-2010/026595** **FR-A1- 2 678 632**
**FR-A1- 2 798 667**

- **anonymous: "ASU Expanscience - Cutting Edge Technology to produce a unique and original ASU", , 13 février 2010 (2010-02-13), pages 1-5, XP002674953, Extrait de l'Internet: URL:http://web.archive.org/web/20100213004455/http://www.original-asu.com/Cutting_Edge__Technology.html [extrait le 2012-04-25]**
- **M. J. WERMAN ET AL: "Avocado oil production and chemical characteristics", JOURNAL OF THE AMERICAN OIL CHEMISTS' SOCIETY, vol. 64, no. 2, 1 février 1987 (1987-02-01), pages 229-232, XP055025527, ISSN: 0003-021X, DOI: 10.1007/BF02542007**
- **"Nahrungsfette und Öle - [Extract] ED - BOCKISCH M", 1 janvier 1993 (1993-01-01), NAHRUNGSFETTE UND -ÖLE, STUTTGART, ULMER, DE, PAGE(S) 73,82,175 - 177, XP009158090, ISBN: 978-3-8001-5817-1 le document en entier**
- **KASHMAN Y ET AL: "New compounds from avocado pear", TETRAHEDRON, ELSEVIER SCIENCE PUBLISHERS, AMSTERDAM, NL, vol. 25, no. 18, 1 janvier 1969 (1969-01-01), pages 4617-4631, XP002090295, ISSN: 0040-4020, DOI: 10.1016/S0040-4020(01)83005-2**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 802 335 B1

- BRIAN I. BROWN: "Isolation of unpleasant flavor compounds in the avocado (Persea americana)", JOURNAL OF AGRICULTURAL AND FOOD CHEMISTRY, vol. 20, no. 4, 1 juillet 1972 (1972-07-01), pages 753-757, XP055025721, ISSN: 0021-8561, DOI: 10.1021/jf60182a019
- GUTFINGER T ET AL: "Studies of unsaponifiables in several vegetable oils", LIPIDS, SPRINGER, US, vol. 9, no. 9, 1 janvier 1974 (1974-01-01) , pages 658-663, XP009158777, ISSN: 0024-4201, DOI: 10.1007/BF02532171
- TZONG-HUEI LEE ET AL: "Heptadecanols from the leaves of Persea americana var. americana", FOOD CHEMISTRY, vol. 132, no. 2, 19 novembre 2011 (2011-11-19), pages 921-924, XP055025945, ISSN: 0308-8146, DOI: 10.1016/j.foodchem.2011.11.067
- Mostert, Mathilda Elizabeth: "Characterization of micro-components of avocado oil extracted with supercritical carbon dioxide and their effect on its oxidative stability", , 15 avril 2008 (2008-04-15), XP002675113, Extrait de l'Internet: URL:http://upetd.up.ac.za/thesis/available /etd-06062008-132406/ [extrait le 2012-05-02]
- BIZIMANA V: "Extraction, characterization, and prediction of the oxidative stability of avocado oil", DISSERTATION, 1997, pages 1-241, XP009158924, University of Minnesota

## Description

La présente description concerne l'utilisation de noyau d'avocat pour obtenir une huile d'avocat enrichie en alkyls polyols et/ou en leurs dérivés acétylés. Avantageusement, ledit noyau d'avocat représente 10 à 50% en masse, en particulier 20 à 40% en masse, par rapport à la masse totale d'avocat utilisé. Les objets de l'invention sont définis dans les revendications. L'invention concerne un procédé d'obtention d'une huile d'avocat enrichie en alkyls polyols et/ou en leurs dérivés acétylés à partir d'au moins des noyaux d'avocats, ladite huile contenant au moins 0,5% en masse d'alkyls polyols et/ou de leurs dérivés acétylés, par rapport à la masse totale de l'huile, lesdits noyaux d'avocat représentant 10 à 50%, en particulier 20 à 40% en masse, par rapport à la masse totale d'avocat utilisé, lesdits avocats utilisés n'étant pas des avocats mous ayant une force de résistance à la pénétration dans la chair inférieure ou égale à 3 $kg/cm^2$, ladite force de résistance étant mesurée à l'aide d'un pénétromètre, tel que défini dans la revendication 1. L'invention concerne également une huile d'avocat enrichie en alkyls polyols et/ou en leurs dérivés acétylés, susceptible d'être obtenue par ce procédé, contenant au moins 0,5% en masse d'alkyls polyols ou de leurs dérivés acétylés, par rapport à la masse totale de l'huile. La présente description divulgue également l'utilisation de l'huile d'avocat pour préparer un concentrât d'huile d'avocat enrichi en alkyls polyols et/ou en leurs dérivés acétylés, ou pour préparer un insaponifiable d'avocat enrichi en alkyls polyols. Enfin, l'invention concerne un insaponifiable d'avocat enrichi en alkyls polyols et/ou en leurs dérivés acétylés ou encore un concentrât d'huile d'avocat enrichi en alkyls polyols et/ou en leurs dérivés acétylés, ledit concentrat contenant au moins 5% d'alkyls polyols et/ou de leur dérivés acétylés en masse, par rapport à la masse totale du concentrât, ledit insaponifiable et ledit concentrat d'huile étant susceptible d'être obtenu à partir de ladite huile d'avocat, pour son utilisation comme médicament, avantageusement dans la prévention et/ou le traitement des troubles du tissu conjonctif tels que l'arthrose, des pathologies articulaires telles que les rhumatismes, ou encore des maladies parodontales, telles que la gingivite ou la parodontitc.

Depuis les années 1990, les débouchés mondiaux de l'avocat se diversifient. Les filières principales restent majoritairement orientées vers l'exportation ou les marchés locaux, en fonction des pays producteurs. Cependant, les volumes destinés à l'industrie sont en forte croissance et sont généralement représentés par la transformation de la pulpe fraîche dans le domaine alimentaire, ainsi que la production d'huile d'avocat de qualité alimentaire ou cosmétique.

Ces dernières applications, si elles restent marginales par rapport aux tonnages dédiés à la commercialisation de l'avocat comme fruit de bouche, permettent de valoriser les écarts de triage et les fruits rejetés par ce marché. Elles représentent une matière première avec un coût d'accès beaucoup plus acceptable.

Ces industries de transformation sont principalement destinées à la valorisation de la pulpe de l'avocat. Elles génèrent de ce fait des sous-produits, résultant du dépulpage des fruits. A titre d'exemple, les filières de production du guacamole et de l'huile d'avocat alimentaire et cosmétique, obtenue par centrifugation, n'utilisent que la pulpe de l'avocat. Ainsi, sont générées des quantités très importantes de coproduits que sont le noyau et la peau du fruit de l'avocat, qui ne sont pas valorisés dans la filière alimentaire.

L'huile d'avocat est en effet stockée principalement dans les cellules de réserve de la pulpe de la pulpe, i.e. idioblastes. Le noyau de l'avocat est, quant à lui, pauvre en huile et ne présente donc que peu d'intérêt pour la filière des huileries.

Des débouchés ont été étudiés et développés pour tenter de valoriser ces sous-produits que sont la peau et le noyau : épandage, mulching, horticulture, alimentation animale, mais ils n'apportent pas de réelle valeur ajoutée.

Cependant, même si les parties constitutives de l'avocat que sont la peau et le noyau sont naturellement pauvres en huile, elles contiennent des composés constitutifs de l'insaponifiable présentant un potentiel actif intéressant et à forte valeur ajoutée.

Néanmoins, ces composés étant en faibles quantités dans ces parties du fruit, telles que le noyau, elles sont de ce fait difficilement accessibles et extractibles.

La forte teneur en eau des différentes parties constitutives des avocats les rend difficiles à travailler et rend presque impossible leur traitement par les procédés physiques d'extraction connus de l'homme du métier dans des conditions économiques acceptables.

Par ailleurs, la faible teneur en huile et en composés actifs dans le noyau d'avocat ne permet pas d'appliquer un traitement physique par pression mécanique, cette technique n'étant pas suffisamment efficace pour traiter des produits présentant des teneurs en huile inférieure à 10%.

Seule l'extraction par solvant apparaît ainsi comme envisageable, mais outre le fait que ce procédé requiert une technologie complexe, il est réputé pour être onéreux, polluant, à impact pour l'homme et l'environnement.

Il existait donc un besoin de trouver un procédé qui permette, à faible coût, de valoriser ces composés actifs potentiellement disponibles dans des coproduits facilement accessibles.

Durant ces dernières décennies, les connaissances de la chimie de l'avocat se sont considérablement enrichies. Plusieurs familles de composés ont ainsi été isolées et identifiées à partir des fruits et de nombreuses études ont été menées pour mettre en évidence leurs activités biologiques. La composition de la fraction insaponifiable de l'huile d'avocat a été particulièrement étudiée.

Les coproduits de l'industrie alimentaire de l'avocat, et notamment le noyau, sont pauvres en huile, mais contiennent tout ou partie des composés constitutifs de l'insaponifiable. En particulier, les composés du type alkyls polyols sont des constituants de l'insaponifiable de l'avocat, et connus pour être particulièrement intéressants dans le traitement de troubles du tissu conjonctif, tels que l'arthrose.

La demande internationale WO 2013/098393 divulgue un procédé de préparation d'huile d'avocat riche en insaponifiables à partir d'avocats mous entiers, lesdits insaponifiables contenant des acétogénines aliphatiques et/ou leurs dérivés. La demande internationale WO 2013/098393 divulgue ainsi un procédé d'obtention d'une huile d'avocat à partir d'avocats mous entiers, ladite huile contenant au moins 3% en masse insaponifiable par rapport à la masse totale de l'huile, dans laquelle ledit insaponifiable contient des acétogénines aliphatiques et/ou leurs dérivés, comprenant les étapes successives suivantes :

(1) Broyage des avocats mous conduisant à l'obtention d'une granulométrie du broyât comprise entre 2 et 20 mm, en particulier entre 2 et 10 mm,
(2) Séchage du broyât à haute température, avantageusement à une température comprise entre 60 et 150°C, en particulier entre 65 et 120°C, par exemple entre 70 et 100°C, typiquement entre 80 et 100°C, jusqu'à l'obtention d'une humidité résiduelle inférieure ou égale à 5%,
(3) Addition d'eau aux avocats séchés par ajout de 1 à 5 % d'eau ou de vapeur d'eau par rapport à la masse des avocats secs broyés, puis
(4) Extraction de l'huile par pression mécanique.

Par ailleurs, le document BIZIMANA V (« Extraction, characterization, and prediction of the oxidative stability of avocado oil », Dissertation, 1997, pages 1-241, University of Minnesota) divulgue des méthodes d'extraction d'huile par solvant, tel que l'hexane, l'éthanol ou l'acétone. En particulier, ce document compare deux méthodes d'extraction par solvant (chloroforme/méthanol versus acétone) des tissus d'avocat.

Il était donc intéressant de trouver un procédé qui permette d'extraire à moindre coût certains composés constitutifs de l'insaponifiable d'avocat, tels que mentionnés ci-dessus, en particulier à partir de co-produits ou sous-produits de la filière alimentaire de l'avocat, tels que les noyaux d'avocat.

La présente invention vient combler ce besoin. La Demanderesse a ainsi découvert un nouveau procédé d'obtention d'une huile d'avocat enrichie en alkyls polyols ou en leurs dérivés acétylés, à partir notamment de noyau d'avocat.

Le procédé selon la présente invention permet ainsi de valoriser les sous-produits de l'avocat, tels que le noyau, et d'extraire avantageusement les composés présents dans le noyau qui ne sont pas extractibles par les procédés classiques d'extraction. De tels composés se trouvent solubilisés, puis extraits par l'huile lors de son extraction dans le cadre du procédé objet de la présente invention, et se trouvent de ce fait valorisés.

Par ailleurs, le procédé selon la présente invention permet, de par l'utilisation de noyau d'avocat en une certaine proportion, d'obtenir une certaine teneur en particules solides dans le produit de départ à extraire, et d'améliorer ainsi l'étape de séchage de l'avocat en facilitant l'élimination de l'eau.

En outre, toujours de par l'utilisation de noyaux d'avocat en une certaine proportion, 10 à 50 % en masse, typiquement 20 à 40 % en masse, par rapport à la masse totale du produit de départ mis en œuvre, avantageusement par l'ajout de noyau aux fruits, le procédé objet de la présente invention permet d'améliorer, de façon significative et inattendue, les performances des procédés classiques d'extraction qui partent généralement de la pulpe d'avocat, dont on a au préalable retiré le noyau. Ainsi, l'utilisation de noyau d'avocat en quantité importante dans le produit de départ permet d'augmenter la charge en matière sèche initiale et ainsi d'améliorer le rendement de pression du procédé en augmentant le taux d'huile récupérée par rapport à la teneur potentielle présente dans la matière entrante, lors de l'étape d'extraction de l'huile.

Par ailleurs, l'augmentation de la proportion de noyau permet, de par ses propriétés mécaniques, un échange entre les différentes fractions en contact dans la matière de départ et l'extraction des composés actifs, ce qui a pour conséquence un enrichissement notable de l'huile en composés insaponifiables spécifiques, tels que les alkyls polyols et leurs dérivés acétylés.

De façon avantageuse selon la présente invention, l'huile extraite majoritairement de la pulpe joue un rôle de vecteur pour solubiliser et extraire les composés insaponifiables spécifiques contenus dans le noyau. De par leur caractère lipophile, ces composés difficilement accessibles au sein de la structure fibreuse du noyau, se trouvent entrainés lors de l'étape d'extraction par pression par le flux d'huile produite qui diffuse dans toute la masse brassée dans le corps de la presse d'extraction.

De façon encore plus avantageuse selon l'invention, il a été mis en évidence le rôle favorable de la température sur le taux d'extraction de ces dérivés lors de l'extraction par pression mécanique de l'huile. Les composés alkyls polyols et/ou leurs dérivés acétylés, de par leur structure moléculaire, possèdent un caractère lipophile, mais la présence de fonctions alcools limite leur affinité pour les corps gras, et de ce fait leur solubilité. L'extraction à haute température

permet ainsi de réduire l'influence de cette propriété et de garantir un niveau de solubilité suffisant pour obtenir un taux d'extraction performant avec le procédé par pression mécanique.

En outre, on peut avantageusement extraire un insaponifiable d'avocat, enrichi en alkyls polyols, qui peut être incorporé au sein de compositions cosmétiques, dermatologiques, pharmaceutiques ou de dispositifs médicaux, ou encore dans des compositions alimentaires, des compléments alimentaires ou des nutraceutiques, à visée humaine ou animale.

La présente description divulgue ainsi l'utilisation de noyau d'avocat pour obtenir une huile d'avocat enrichie en alkyls polyols et/ou en leurs dérivés acétylés, ledit noyau d'avocat représentant 10 à 50% en masse par rapport à la masse totale d'avocat utilisé. De manière particulièrement avantageuse, le noyau d'avocat représente 15 à 40%, typiquement 20 à 40%, en masse par rapport à la masse totale d'avocat utilisé.

En particulier, la présente description divulgue l'utilisation de noyau d'avocat pour obtenir par pression mécanique une huile d'avocat enrichie en alkyls polyols et/ou en leurs dérivés acétylés, ledit noyau d'avocat représentant 10 à 50% en masse par rapport à la masse totale d'avocats utilisés, lesdits alkyls polyols étant des triols du type 1,2,4-trihydroxy à chaînes aliphatiques non ramifiées en C17 à C21, saturées, mono ou polyinsaturées.

De manière particulièrement avantageuse, on utilise de la pulpe d'avocat conjointement au noyau d'avocat dans le cadre de la présente invention. Typiquement, la pulpe de l'avocat représente au moins 50% en masse par rapport à la masse totale d'avocat utilisé.

La présente description divulgue aussi l'utilisation de l'huile d'avocat contenue dans la pulpe de l'avocat pour extraire les composés alkyls polyols et/ou leurs dérivés acétylés du noyau, la pulpe de l'avocat représentant au moins 50% en masse par rapport à la masse totale d'avocat utilisé.

La plupart des variétés d'avocat peuvent être utilisées dans le cadre de la présente invention pour produire de l'huile, du concentrat puis l'insaponifiable d'avocat, avec les caractéristiques recherchées, dans la mesure où elles renferment le potentiel qualitatif et quantitatif en composés spécifiques.

De manière particulièrement avantageuse, le procédé selon l'invention est appliqué aux variétés les plus cultivées et représentant la *quasi* totalité des tonnages exportés et commercialisés au niveau mondial, préférentiellement les variétés *hass* et *fuerte.*

Dans un mode de réalisation particulier selon la présente invention, les avocats utilisés comme produit de départ sont des avocats entiers, auxquels on peut ajouter avantageusement des noyaux. De manière avantageuse, on utilise ainsi comme mélange de départ des avocats entiers auxquels on ajoute des noyaux.

Par le terme d'avocats « entiers », on entend, au sens de la présente invention, des avocats contenant la peau, la pulpe et le noyau distribués dans leur intégrité.

Dans un autre mode de réalisation particulier selon la présente invention, les avocats utilisés comme produit de départ sont des avocats sans peau, auxquels on peut ajouter avantageusement des noyaux. De manière avantageuse, on utilise ainsi comme mélange de départ des avocats sans peau auxquels on ajoute des noyaux.

Par le terme d'avocats « sans peau », on entend, au sens de la présente invention, des avocats entiers dans lesquels la peau a été retirée, et ne contenant ainsi plus que le noyau et la pulpe.

Dans un autre mode de réalisation particulier selon la présente invention, les avocats utilisés comme produit de départ sont de la pulpe d'avocat à laquelle on ajoute des noyaux.

Dans le cadre de la présente invention, l'ajout de noyau aux avocats avec ou sans peau, ou encore à la pulpe d'avocat, est particulièrement avantageux, puisqu'il permet ainsi d'augmenter la proportion de noyau par rapport aux différentes parties du fruit dans le mélange de départ mis en œuvre avant d'opérer les étapes de séchage des avocats et d'extraction de l'huile. Cela permet d'améliorer les étapes subséquentes de séchage et d'extraction de l'huile, notamment d'extraction par pression mécanique en augmentant significativement le rendement de pression.

De façon encore plus particulière selon l'invention, il a été montré que l'augmentation de la proportion de noyau par rapport aux différentes parties du fruit dans le mélange de départ devait être accompagnée d'une addition d'eau ou de vapeur d'eau en amont de la pression pour obtenir un rendement de pression optimal.

Avantageusement selon l'invention, l'apport conjoint de noyau dans la masse pour structurer la matrice travaillée dans la presse d'extraction, puis d'eau ou de vapeur d'eau, génère un flux gazeux et une augmentation de pression au sein du produit qui favorise l'éclatement des cellules, la libération de l'huile, sa diffusion dans la masse et son expulsion hors de la cage de la presse.

Selon une caractéristique particulière de la description, les avocats utilisés sont des avocats mous.

Typiquement, les avocats mous ont un degré de ramollissement équivalent à celui d'une consommation immédiate de l'avocat, et excluent un prétraitement par tranchage.

Avantageusement, les avocats mous sont caractérisés par la consistance de leur chair mesurée à l'aide d'un pénétromètre et définie par une force de résistance à la pénétration. De manière particulièrement avantageuse, les avocats mous ont une force de résistance à la pénétration dans la chair inférieure ou égale à 3 kg/$cm^2$, typiquement inférieure ou égale à 2 kg/$cm^2$, par exemple inférieure ou égale à 1 kg/$cm^2$.

Selon la présente invention, les avocats utilisés ne sont pas des avocats mous. Plus particulièrement, selon

un mode de réalisation de l'invention, les avocats utilisés sont des avocats durs.

Selon la présente invention, les avocats durs ont une force de résistance à la pénétration dans la chair supérieure à 3 $kg/cm^2$.

Typiquement selon la présente invention, la force de pénétration est mesurée à l'aide d'un pénétromètre du type PCE-PTR 200 ou FT 327, qui mesure la force en kilogramme nécessaire pour faire pénétrer un embout calibré dans le fruit. Avantageusement, le fruit est pelé avant de faire la mesure afin de s'affranchir de la résistance de la peau (tégument) et de la variabilité des différentes variétés d'avocat testées. La tige (pointal ou embout) utilisée pour cette mesure a un diamètre nominal respectivement de 6 et de 11,3 mm.

Selon l'invention, les avocats utilisés sont broyés ou tranchés, puis séchés à haute température, entre 60 et 150°C, jusqu'à l'obtention d'une humidité résiduelle inférieure ou égale à 5%, avant l'obtention de l'huile par pression mécanique.

Selon la présente invention, suite au broyage et au séchage des avocats, 1 à 5 % d'eau ou de vapeur d'eau, par rapport à la masse des avocats secs, est additionné avant l'obtention de l'huile par pression mécanique.

Il a en effet été découvert que lorsque l'on intègre une étape d'injection d'eau ou de vapeur d'eau au sein des avocats séchés, ceci permet d'obtenir une huile d'avocat enrichie en alkyls polyols et/ou en leurs dérivés acétylés avec un rendement élevé.

L'huile selon l'invention est enrichie en alkyls polyols et/ou en leurs dérivés acétylés.

Par le terme d'huile « enrichie en alkyls polyols et/ou en leurs dérivés acétylés », on entend au sens de la présente invention une huile contenant au moins 0,5% en masse d'alkyls polyols et/ou de leurs dérivés acétylés, par rapport à la masse totale de l'huile. En particulier, l'huile contient entre 0,5 et 10% en masse d'alkyls polyols et/ou de leurs dérivés acétylés, par exemple entre 1 et 8%, plus particulièrement entre 1,5 et 6%, en masse d'alkyls polyols et/ou de leurs dérivés acétylés, par rapport à la masse totale de l'huile.

Dans le cadre de l'invention, les alkyls polyols, encore appelés alcools gras polyhydroxylés, sont notamment des triols du type 1,2,4-trihydroxy à chaînes aliphatiques non ramifiées en C17 à C21, saturées, mono ou polyinsaturées, par exemple à longues chaînes acétyléniques et oléfiniques, avantageusement choisis dans le groupe constitué par le nonadecane-1,2,4-triol, le heneicosa-cis, cis-12-15 diene-1,2,4-triol; le heptadec-16-yne-1,2,4-triol, le heptadec-cis-16-ene-1,2,4-triol, et leurs mélanges.

Selon une caractéristique particulière de la présente invention, les dérivés acétylés des alkyls polyols sont des composés mono-, di, ou tri-acétylés, de préférence mono-acétylés, typiquement en position 1, 2 ou 4.

Typiquement, l'huile d'avocat selon l'invention contient des stérols et/ou des hydrocarbures aliphatiques saturés.

Les stérols sont notamment des hydrocarbures tétracycliques comportant une fonction alcool en position 3 et une double liaison dont la position intracyclique est majoritairement en position 5.

Dans le cadre de l'invention, les stérols sont avantageusement choisis dans le groupe constitué par le β-Sitostérol, le Campestérol, le Stigmastérol, le Δ5-Avenastérol, le Δ7-Stigmastérol, le Citrostadiénol, et leurs mélanges.

Selon une caractéristique particulière de l'invention, l'huile contient au moins 0,5% en masse de stérols, avantageusement au moins 0,8% en masse de stérols, par rapport à la masse totale de l'huile.

Dans le cadre de l'invention, les hydrocarbures aliphatiques saturés sont notamment des hydrocarbures à chaîne linéaire non ramifiée présentant un nombre impair de carbones. Avantageusement, les hydrocarbures aliphatiques saturés sont des alcanes en $C_{27}$, $C_{29}$ ou $C_{31}$.

En particulier, les hydrocarbures aliphatiques saturés selon l'invention sont choisis dans le groupe constitué par le n-heptacosane ($CH_3(CH_2)_{25}CH_3$), le n-nonacosane ($CH_3(CH_2)_{27}CH_3$), le n-hentriacontane ($CH_3(CH_2)_{29}CH_3$), et leurs mélanges.

Selon une caractéristique particulière de l'invention, l'huile contient au moins 0,01% en masse d'hydrocarbures aliphatiques saturés, avantageusement entre 0,01 et 0,10% en masse, par rapport à la masse totale de l'huile.

Avantageusement, l'huile d'avocat selon l'invention contient des 1,2-dihydroxy-4-oxo-aliphatiques alcools et/ou leurs dérivés acétylés de type «persins» et/ou des alkyls furanes.

Les 1,2-dihydroxy-4-oxo-aliphatiques alcools sont encore appelés persins, et sont des précurseurs des alkyl furanes. Il s'agit notamment des diols cétones du type 1-2-dihydroxy-4-oxo à longues chaînes saturées ou acétyléniques ou oléfiniques. Les dérivés acétylés de ces composés sont typiquement en position 1.

Les persins se trouvent typiquement dans les idioblastes, cellules oléagineuses dans le cas de l'avocat.

Par exemple, on peut citer particulièrement les persins de structure moléculaire suivante :

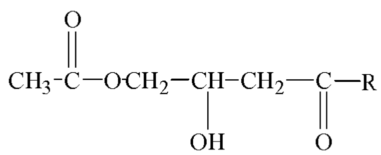

Les alkyls furanes ou furanes aliphatiques sont encore appelés lipides furaniques ou plus communément avocadofuranes. Il s'agit notamment de dérivés des persins comportant un groupement furane, qui résultent en particulier de la transformation chimique par déshydratation et cyclisation intramoléculaire des persins extraites de l'avocat. A titre d'exemple, on peut citer les 2-alkyls furanes.

Avantageusement, l'huile d'avocat selon l'invention contient au moins 2% en masse, plus avantageusement au moins 3% en masse, par exemple entre 3 et 12% en masse de 1,2-dihydroxy-4-oxo-aliphatiques alcools et/ou leurs dérivés acétylés de type «persins» et/ou d'alkyls furanes, par rapport à la masse totale de l'huile.

La présente description divulgue également un procédé d'obtention d'une huile d'avocat enrichie en alkyls polyols et/ou en leurs dérivés acétylés, à partir d'au moins des noyaux d'avocats, lesdits noyaux d'avocat représentant 10 à 50% en masse par rapport à la masse totale d'avocats utilisés, comprenant les étapes successives suivantes :

- Tranchage ou broyage d'avocats contenant une teneur en noyaux de 10 à 50% m/m,
- Séchage à haute température, avantageusement à une température comprise entre 60 et 150°C, en particulier entre 80 et 120°C, puis
- Extraction de l'huile.

La présente invention a pour objet un procédé d'obtention d'une huile d'avocat enrichie en alkyls polyols et/ou en leurs dérivés acétylés, à partir d'au moins des noyaux d'avocats, ladite huile contenant au moins 0,5% en masse d'alkyls polyols et/ou de leurs dérivés acétylés, lesdits noyaux d'avocat représentant 10 à 50% en masse par rapport à la masse totale d'avocats utilisés, lesdits avocats utilisés n'étant pas des avocats mous ayant une force de résistance à la pénétration dans la chair inférieure ou égale à 3 $kg/cm^2$, ladite force de résistance étant mesurée à l'aide d'un pénétromètre, et ledit procédé comprenant les étapes successives suivantes :

(1) Tranchage ou broyage d'avocats contenant une teneur en noyaux de 10 à 50% m/m, ledit broyage conduisant à l'obtention d'une granulométrie de broyat comprise entre 2 et 20 mm,
(2) Séchage à haute température comprise entre 60 et 150°C, en particulier entre 80 et 120°C, jusqu'à l'obtention d'une humidité résiduelle inférieure ou égale à 5%.
(3) Addition d'eau aux avocats séchés par ajout de 1 à 5% d'eau ou de vapeur d'eau par rapport à la masse des avocats secs, puis avantageusement homogénéisation par malaxage, avant introduction dans la presse, puis
(4) Extraction de l'huile par pression mécanique, avantageusement à une température comprise entre 80 et 100°C.

Avantageusement selon la présente invention, les alkyls polyols sont des triols du type 1,2,4-trihydroxy à chaînes aliphatiques non ramifiées en C17 à C21, saturées, mono ou polyinsaturées, avantageusement choisis dans le groupe constitué par le nonadecane-1,2,4-triol; le heneicosa-cis, cis-12-15 diene-1,2,4-triol, le heptadec-16-yne-1,2,4-triol ; le heptadec-cis-16-ene-1,2,4-triol ; et leurs mélanges.

Dans un mode de réalisation particulier du procédé selon la présente invention, les avocats utilisés comme produit de départ sont des avocats entiers, auxquels on peut ajouter avantageusement des noyaux. De manière avantageuse, on utilise ainsi comme mélange de départ des avocats entiers auxquels on ajoute des noyaux.

Les avocats entiers sont tels que définis ci-dessus.

Dans un autre mode de réalisation particulier du procédé selon la présente invention, les avocats utilisés comme produit de départ sont des avocats sans peau, auxquels on peut ajouter avantageusement des noyaux. De manière avantageuse, on utilise ainsi comme mélange de départ des avocats sans peau auxquels on ajoute des noyaux.

Les avocats sans peau sont tels que définis ci-dessus.

Dans un autre mode de réalisation particulier du procédé selon la présente invention, les avocats utilisés comme produit de départ sont de la pulpe d'avocat à laquelle on ajoute des noyaux.

Avantageusement selon l'invention, les avocats utilisés comme produit de départ ont une teneur en noyau comprise entre 15 et 40% en masse, en particulier 20 à 40% en masse, par rapport à la masse totale d'avocat utilisé.

Dans un mode de réalisation particulier selon la présente invention, le broyage (1) est réalisé sur les avocats entiers constitués de la peau, de la pulpe et du noyau, ou encore sur les avocats sans peau constitués du noyau et de la pulpe.

Le broyage (1) permet avantageusement de déchiqueter la peau, de morceler le noyau et de malaxer le mélange afin d'obtenir une dispersion et une granulométrie homogènes du broyat (particules et morceaux obtenus) dans la pulpe d'avocat.

Typiquement, les broyeurs utilisés s'adaptent à la très grande différence de texture et de dureté des différentes parties qui composent l'avocat : peau, pulpe et noyau. Ainsi, la technologie de broyeurs à mettre en œuvre doit permettre de traiter des matériaux présentant des parties très dures (noyau) et des parties plus tendres (peau), voire très molles (pulpe).

Le broyage (1) est avantageusement réalisé à l'aide d'un broyeur de type à couteaux ou à rouleaux dentelés.

La configuration et le réglage doivent cependant être généralement adaptés en fonction de la taille des fruits, de leur maturité et de leur qualité (biométrie entre noyau, pulpe et peau) pour aboutir à la granulométrie avantageusement recherchée.

Le broyage (1) est réalisé de telle manière à obtenir une taille spécifique des particules et morceaux, répondant à une fourchette de granulométrie qui donne au broyat une texture adaptée à un séchage rapide. Cette texture se caractérise par un aspect visuel non continu, les morceaux étant détectables généralement dans la masse sans utilisation d'un instrument optique complémentaire. Typiquement, la surface du broyat n'est pas lisse et comporte des aspérités représentées par les particules de noyau et de peau. Lors du séchage, le broyat ne forme pas une masse compacte, mais des blocs facilement friables.

Selon l'invention, le broyage (1) conduit à l'obtention d'une granulométrie du broyat comprise entre 2 et 20 mm, en particulier entre 2 et 10 mm.

De manière avantageuse selon l'invention, le mélange, une fois broyé, doit être réparti de façon adaptée pour garantir une homogénéité du séchage (2) qui suit avec la plus grande efficacité, avantageusement par étalement en couche mince, typiquement pour conduire à un film de faible épaisseur avantageusement comprise entre 0,5 et 5 cm, en particulier entre 1 et 2 cm, ou encore par mise en forme permettant d'optimiser la surface d'évaporation du type extrusion ou passage dans une filière.

Selon une autre caractéristique de l'invention, les avocats utilisés sont des avocats durs. Les avocats durs sont tels que définis ci-dessus.

Dans ce cas, la première étape du procédé selon l'invention consiste en un tranchage des avocats durs.

Le tranchage (1) des avocats est typiquement opéré à l'aide d'une trancheuse à disque.

Le tranchage (1) des avocats conduit avantageusement à des tranches d'épaisseur de 2 à 5 mm.

Les tranches sont ensuite réparties de façon homogène sur des plateaux ou claies de séchage.

L'étape suivante de séchage (2) a pour but d'extraire l'eau du milieu, mais aussi de rendre extractibles les composés polaires de l'insaponifiable. Elle est réalisée, en particulier, grâce à une technologie spécifique et à une température choisie pour optimiser les besoins en énergie et limiter les réactions indésirables. En effet, une température trop basse limiterait la vitesse d'évaporation de l'eau et favoriserait l'action des lipases, entraînant l'hydrolyse des glycérides et l'augmentation de l'indice d'acide du milieu. Une température trop élevée favoriserait le phénomène de croûtage, ainsi que la dégradation thermique et/ou oxydative ou non (réaction de Maillard) des composés sensibles de l'insaponifiable ou des composés insaturés de l'huile.

Il a ainsi été découvert qu'il était recommandé d'utiliser une étape de séchage à température ménagée et contrôlée. La teneur en eau très élevée de l'avocat (≈ 75%) requiert ainsi une technique de séchage très efficace et spécifique pour garantir une évaporation rapide n'induisant pas de dégradation des constituants propres du fruit.

Selon l'invention, le séchage (2) est réalisé à température ménagée et contrôlée comprise entre 60 et 150°C, en particulier entre 65 et 120°C, par exemple entre 80 et 120°C ou entre 70 et 100°C, typiquement entre 80 et 100°C.

Selon une caractéristique particulière de l'invention, le séchage (2) est réalisé pendant 8 à 78 h, avantageusement pendant 10 à 24 h.

Le séchage (2) selon l'invention peut être réalisé en particulier par séchage sous courant d'air chaud ou sous atmosphère contrôlée (ex. azote), par séchage à pression atmosphérique ou sous vide, ou par séchage par micro-ondes.

Dans le cadre du présent procédé, pour des raisons de facilité de mise en œuvre industrielle et pour des raisons de coût, le séchage en séchoirs ventilés, en couche mince et sous courant d'air chaud, à une température comprise entre 80 et 100°C, pendant 8 à 36 heures est préféré.

A l'issue du séchage (2), le produit séché présente un taux d'humidité résiduelle inférieure ou égale à 5% m/m.

L'humidité résiduelle est typiquement mesurée à l'aide d'une méthode thermogravimétrique par séchage IR. D'autres méthodes peuvent également être mises en œuvre, telles que la méthode de perte au séchage en étuve ou la méthode de titrage Karl Fischer.

Une humidité résiduelle inférieure ou égale à 5% joue un rôle important dans la consistance des avocats séchés, leur conférant une texture solide cassante favorable pour résister aux contraintes physiques développées pendant la pression mécanique. Au-delà de 5% d'humidité, l'avocat séché présente une consistance molle qui conduit, lors de la pression, à la formation d'une purée sans consistance suffisante pour être pressée efficacement.

Il a été trouvé que, même avec une humidité résiduelle inférieure ou égale à 5 % et une consistance propice à la pression, ces conditions ne sont pas totalement suffisantes pour atteindre des conditions de pression permettant un rendement d'extraction de l'huile élevé.

Il a ainsi été découvert de façon surprenante qu'un réajustement (3) de l'humidité résiduelle par addition de 1 à 5% d'eau ou de vapeur d'eau, par exemple de 1 à 3 % d'eau ou de vapeur d'eau, par rapport à la masse des avocats secs, permettait d'accroitre considérablement le taux d'extraction de l'huile, et de ce fait l'efficacité de la pression.

Cette opération de réajustement/addition d'eau (3) doit être réalisée juste avant l'introduction des fruits séchés dans la presse par ajout d'eau purifiée ou de vapeur d'eau pour que les avocats secs se saturent en humidité sans perdre leur consistance ferme et afin qu'aucun ramollissement n'apparaisse.

Ce taux d'humidité résiduelle pour être efficace ne peut être obtenu que par déshydratation préalable (2) des avocats, puis réajustement par addition d'eau (3). En effet, une déshydratation partielle contrôlée directe n'aboutit pas à une texture de produit compatible avec la pression, comme il a été signalé précédemment.

De façon particulière selon l'invention, l'addition d'eau (3) dans les avocats séchés est réalisée par ajout contrôlé d'eau ou de vapeur d'eau sur les avocats séchés, puis homogénéisation par malaxage typiquement dans un mélangeur planétaire, avantageusement pendant 1/2h à 1h.

De façon plus particulière selon l'invention, l'addition d'eau (3) dans les avocats séchés est réalisée en continu dans un convoyeur du type vis sans fin. L'eau ou la vapeur d'eau est ajoutée sur les avocats en tête de convoyeur et l'homogénéisation est obtenue par agitation dans le convoyeur, lors du déplacement du produit. Le dimensionnement du convoyeur doit permettre de garantir typiquement un temps de séjour minimum de 1/2h des avocats séchés.

De façon encore plus particulière selon l'invention, le convoyeur est utilisé pour alimenter la presse d'extraction.

L'étape d'extraction (4) de l'huile est ainsi précédée d'une étape d'addition d'eau (3) dans les avocats séchés par ajout de 1 à 5%, de préférence de 1 à 3%, d'eau ou de vapeur d'eau par rapport à la masse des avocats séchés.

L'étape d'addition d'eau (3) dans les avocats séchés selon l'invention est généralement précédée par un broyage du produit séché qui homogénéise le produit d'alimentation.

L'étape d'extraction (4) de l'huile est mise en œuvre par une pression mécanique de la matière sèche, avantageusement après mise œuvre de l'étape d'addition d'eau (3).

Usuellement, pour pouvoir travailler avec efficacité, les presses d'extraction doivent recevoir une matière contenant une teneur en fibres et matière organique adaptée conférant au tourteau produit une certaine consistance. Cette consistance permet d'atteindre, en tête de presse, une pression élevée indispensable pour garantir un rendement de pression adapté.

Avantageusement, la présence de particules de noyau à l'intérieur du mélange lui donne une consistance et une texture favorable à l'extraction de l'huile par pression et améliore de ce fait la productivité et la production de l'huile par pression.

Avantageusement, l'étape d'extraction (4) est réalisée à une température comprise entre 80 et 100°C. Cette température est obtenue et maintenue constante par tous les moyens à disposition tels que, préchauffage du corps du presse, préchauffage des avocats séchés puis additionnés d'eau en alimentation de la presse, chauffage du corps de presse...

En particulier, le maintien de la température de travail de la presse à une valeur supérieure à 80°C, plus particulièrement à 100°C, permet de garantir un bon niveau d'extraction de l'huile et d'améliorer notablement le rendement d'extraction des constituants de l'insaponifiable, notamment les alkyls polyols et/ou en leurs dérivés acétylés.

L'étape d'extraction (4) selon l'invention est généralement complétée par une filtration qui élimine les particules solides et garantit la limpidité de l'huile produite.

Le procédé selon la présente invention permet de produire ainsi une huile d'avocat de qualité avec une composition particulière, notamment possédant un indice d'acide faible et un potentiel élevé en insaponifiable et une composition de cet insaponifiable particulière.

La présente invention a ainsi également pour objet une huile d'avocat enrichie en alkyls polyols et/ou en leurs dérivés acétylés, susceptible d'être obtenue par le procédé selon l'invention.

L'huile contient au moins 0,5% en masse d'alkyls polyols et/ou de leurs dérivés acétylés, par rapport à la masse totale de l'huile.

En particulier, l'huile contient entre 0,5 et 10% en masse d'alkyls polyols et/ou de leurs dérivés acétylés, par exemple entre 1 et 8%, plus particulièrement entre 1,5 et 6%, en masse d'alkyls polyols et/ou de leurs dérivés acétylés, par rapport à la masse totale de l'huile.

Les alkyls polyols, ainsi que leurs dérivés acétylés, contenus dans l'huile sont tels que définis ci-dessus.

Typiquement, l'huile d'avocat selon l'invention contient des stérols et/ou des hydrocarbures aliphatiques saturés.

Les stérols sont avantageusement tels que définis ci-dessus.

Selon une caractéristique particulière de l'invention, l'huile contient au moins 0,5% en masse de stérols, avantageusement au moins 0,8% en masse de stérols, par rapport à la masse totale de l'huile.

Par ailleurs, les hydrocarbures aliphatiques saturés sont avantageusement tels que définis ci-dessus.

Selon une caractéristique particulière de l'invention, l'huile contient au moins 0,01% en masse d'hydrocarbures aliphatiques saturés, avantageusement entre 0,01 et 0,10% en masse, par rapport à la masse totale de l'huile.

Avantageusement, l'huile d'avocat selon l'invention contient des 1,2-dihydroxy-4-oxo-aliphatiques alcools et/ou leurs dérivés acétylés de type «persins» et/ou des alkyls furanes.

Les 1,2-dihydroxy-4-oxo-aliphatiques alcools, leurs dérivés acétylés, ainsi que les alkyls furanes, sont avantageusement tels que définis ci-dessus.

Avantageusement, l'huile d'avocat selon l'invention contient au moins 2% en masse, plus avantageusement au moins 3% en masse, par exemple entre 3 et 12% en masse de 1,2-dihydroxy-4-oxo-aliphatiques alcools et/ou leurs dérivés acétylés de type «persins» et/ou d'alkyls furanes, par rapport à la masse totale de l'huile.

De manière particulièrement avantageuse, l'huile selon l'invention présente un faible indice d'acide, généralement inférieur ou égal à 5 mg KOH/g, avantageusement inférieur ou égal à 4 mg KOH/g, typiquement inférieur ou égal à 3 mg KOH/g.

La présente description divulgue également une composition contenant une huile d'avocat enrichie en alkyls polyols et/ou en leurs dérivés acétylés, avantageusement à une concentration comprise entre 0,1 et 99,9% en masse, encore plus avantageusement de 30 à 70% en masse, par rapport à la masse totale de la composition.

La présente description divulgue également l'utilisation de l'huile d'avocat telle que définie ci-dessus pour préparer un concentrat d'huile d'avocat enrichi en alkyls polyols et/ou en leurs dérivés acétylés.

Par le terme de concentrat d'huile d'avocat enrichi en alkyls polyols et/ou en leurs dérivés acétylés, on entend au sens de la présente invention un concentrat d'huile d'avocat contenant une teneur élevée en insaponifiable, en particulier en alkyls polyols ou en leurs dérivés, contenant au moins 5%, par exemple entre 10 et 30% d'alkyls polyols et/ou de leurs dérivés acétylés en masse, par rapport à la masse totale du concentrat.

La présente description divulgue également l'utilisation de l'huile d'avocat telle que définie ci-dessus ou du concentrat d'huile d'avocat tel que mentionné ci-dessus pour préparer un insaponifiable d'avocat enrichi en alkyls polyols.

Les étapes de concentration de l'huile en sa fraction insaponifiable pour préparer un concentrat tel que mentionné ci-dessus, et de préparation d'un insaponifiable d'avocat enrichi en alkyls polyols à partir de l'huile ou du concentrat sont en particulier celles décrites ci-dessous.

La préparation du concentrat est généralement opérée par cristallisation à froid ou par distillation moléculaire. Avantageusement, le concentrat en insaponifiable d'huile d'avocat est préparé par distillation moléculaire, typiquement à une température comprise entre 180 et 260°C, en maintenant une pression comprise entre $10^{-2}$ et $10^{-3}$ mmHg.

Cette étape de distillation moléculaire de l'huile d'avocat est de préférence réalisée en utilisant un dispositif choisi parmi les distillateurs moléculaires de type centrifuge et les dispositifs de type film raclé.

La préparation de l'insaponifiable d'avocat enrichi en alkyls polyols à partir de l'huile ou du concentrat inclut généralement un traitement thermique de l'huile ou du concentrat à une température comprise entre 80 et 150°C, par exemple entre 80 et 130°C, suivie d'une étape de saponification et d'extraction de l'insaponifiable, par exemple à l'aide d'un solvant.

Le procédé de préparation de l'insaponifiable d'avocat enrichi en alkyls polyols comprend en particulier une étape de saponification et d'extraction de l'insaponifiable, par exemple à l'aide d'un solvant.

En particulier, l'étape (5) de saponification et d'extraction de l'insaponifiable peut être mise en œuvre en présence de potasse ou de soude en milieu alcoolique, de préférence éthanolique, suivie d'une ou plusieurs extraction(s). L'extraction par un solvant organique approprié (extraction liquide-liquide) en vue de séparer les savons d'acides gras et les composés insaponifiables, est particulièrement adaptée. Le solvant organique approprié peut être, par exemple choisi dans le groupe des alcanes, des alcanes halogénés, des solvants aromatiques et aromatiques halogénés, des éthers, des cétones, des esters, des solvants comprenant au moins un atome de silicium, ou tout autre solvant approprié non miscible avec la solution hydro-alcoolique.

Suite à l'extraction de l'insaponifiable, on peut procéder à des étapes complémentaires de purification ou de fractionnement.

La présente invention a également pour objet le concentrat d'huile d'avocat enrichi en fraction insaponifiable préparé à partir de l'huile d'avocat selon l'invention, ledit concentrat contenant au moins 5% d'alkyls polyols et/ou de leurs dérivés acétylés en masse, par rapport à la masse totale du concentrat, pour son utilisation comme médicament.

La présente description divulgue également l'insaponifiable total ou la fraction insaponifiable préparé(e) à partir de l'huile d'avocat selon l'invention ou du concentrat d'huile d'avocat selon l'invention.

La présente description divulgue également un insaponifiable d'avocat enrichi en alkyls polyols, susceptible d'être obtenu par le procédé selon l'invention.

En particulier, l'insaponifiable d'avocat selon la description contient des hydrocarbures aliphatiques saturés et des stérols.

La présente description divulgue également une composition contenant un insaponifiable selon l'invention tel que mentionné ci-dessus, avantageusement à une concentration comprise entre 0,1 et 99,9% en masse, encore plus avantageusement de 30 à 70% en masse, par rapport à la masse totale de la composition.

Ladite composition peut, en outre, comprendre d'autres actifs.

Parmi les actifs recommandés en association avec l'insaponifiable selon l'invention, on peut citer les extraits végétaux, en particulier :

- Les huiles ou beurres végétaux tels que les huiles de Soja et/ou l'huile de Colza, l'huile de Lupin, avantageusement l'huile de Lupin blanc doux, ou un mélange de ces huiles ou beurres ;
- l'oléodistillat ou les concentrats d'huile végétale ou animale, notamment de tournesol, plus avantageusement des concentrats de Tournesol linoléiques, tels que l'huile de tournesol concentrée en insaponifiable (Soline®) commercialisée par les Laboratoires Expanscience, les huiles concentrées en insaponifiable du type huile de soja, de colza,

de maïs ou de palme ;

- les insaponifiables de végétaux ou d'huile végétale, avantageusement des furanes d'avocat (Avocadofurane®), les insaponifiables d'Avocat et/ou de Soja, plus particulièrement un mélange d'insaponifiables d'Avocat furaniques et d'insaponifiables de Soja, avantageusement dans un rapport respectif d'environ 1/3-2/3 (tel que Piasclédine®300), les insaponifiables de soja, les insaponifiables stéroliques (typiquement des insaponifiables dont la teneur globale en stérols, en méthylstérols et en alcools triterpèniques, est comprise entre 20 et 95 % en masse, de préférence 45-65 % en masse, par rapport à la masse totale de l'insaponifiable), les phytostérols, les esters de stérols et les dérivés vitaminiques.

En particulier, la composition selon l'invention contient un insaponifiable d'avocat, selon l'invention, en association avec un insaponifiable de soja, avantageusement dans un rapport d'environ 2/3 pour le soja et 1/3 pour l'avocat (tel que Piasclédine®300).

Dans un mode de réalisation particulier de la description, la composition comprend en outre au moins un composé choisi parmi les composés suivants, connus pour leurs propriétés dans le traitement du tissu conjonctif, en particulier dans le traitement de l'arthrose :
les sucres aminés tels que la glucosamine ; les sels de glucosamine, tels que le chlorhydrate de glucosamine (par exemple de 1500 à 2000 mg/j), le sulfate de glucosamine, le phosphate de glucosamine et le N-acétylglucosamine ; les glycosaminoglycanes (GAG) tels que la chondroïtine sulfate (par exemple de 800 à 1200 mg/j) ; les analogues de glycosaminoglycanes tels que les glycosaminoglycanes polysulfatés, ou les précurseurs de glycosaminoglycanes tels que l'acide hyaluronique, l'acide glucuronique, l'acide iduronique, le keratane sulfate, le heparane sulfate, ou le dermatine sulfate ; la pentosane ou ses dérivés, en particulier la pentosane sulfate, la pentosane polysulfate (PPS) et les polysaccharides pentosane polysulfates ; la S-adénosylméthionine (SAMe) ; l'adénosine ; la superoxyde dismutase (SOD) ; la L-ergothionine ; les collagènes de type II hydrolysés ou non ; les hydrolysats de collagène tels que la gélatine ; la diacérine ; l'acide arachidonique ; la tétracycline ; les composés analogues de la tétracycline ; la doxycycline ; l'hydroxyproline ; et leurs mélanges.

Avantageusement, la composition comprend en association plusieurs des composés mentionnés ci-dessus. La glucosamine et la chondroïtine sulfate, seuls ou en association, sont des composés particulièrement préférés.

Dans un autre mode de réalisation particulier de la description, la composition comprend en outre au moins un anti-inflammatoire non stéroïdien (AINS), tel que le paracétamol.

Enfin, la présente invention a également pour objet l'insaponifiable d'avocat enrichi en alkyls polyols, tel que décrit ci-dessus, pour son utilisation comme médicament, à visée humaine ou animale, avantageusement dans la prévention et/ou le traitement des troubles du tissu conjonctif tels que l'arthrose, des pathologies articulaires telles que les rhumatismes, des maladies parodontales, telles que la gingivite ou la parodontite.

En particulier, la présente invention a pour objet un insaponifiable d'avocat enrichi en alkyls polyols, susceptible d'être obtenu selon le procédé de l'invention, pour son utilisation comme médicament, avantageusement dans la prévention et/ou le traitement des troubles du tissu conjonctif tels que l'arthrose, des pathologies articulaires telles que les rhumatismes, ou encore des maladies parodontales, telles que la gingivite ou la parodontite.

La présente description divulgue également la composition telle que décrite ci-dessus, pour son utilisation comme médicament, à visée humaine ou animale, avantageusement dans la prévention et/ou le traitement des troubles du tissu conjonctif tels que l'arthrose, des pathologies articulaires telles que les rhumatismes, des maladies parodontales, telles que la gingivite ou la parodontite ou encore dans la prévention et/ou le traitement des troubles du derme et/ou de l'hypoderme tels que le vieillissement cutané, les vergetures et la cellulite, ou encore des troubles de la barrière épidermique tels que les inflammations cutanées, l'eczéma atopique et les dermatites irritatives et/ou inflammatoires.

Il est également décrit l'insaponifiable d'avocat enrichi en alkyls polyols, tel que décrit ci-dcssus ou la composition telle que décrite ci-dcssus, pour son utilisation comme dispositif médical, comme agent dermatologique, comme agent cosmétique, ou comme nutraceutique, avantageusement dans la prévention et/ou le traitement des troubles du tissu conjonctif tels que l'arthrose, des pathologies articulaires telles que les rhumatismes, des maladies parodontales, telles que la gingivite ou la parodontite ou encore dans la prévention et/ou le traitement des troubles du derme et/ou de l'hypoderme tels que le vieillissement cutané, les vergetures et la cellulite, ou encore des troubles de la barrière épidermique tels que les inflammations cutanées, l'eczéma atopique et les dermatites irritatives et/ou inflammatoires.

Par ailleurs, on entend par dispositif médical selon la description tout instrument, appareil, équipement, matière, produit, à l'exception des produits d'origine humaine, ou autre article seul ou en association, destiné par le fabricant à être utilisé chez l'homme à des fins médicales et dont l'action principale voulue n'est pas obtenue par des moyens pharmacologiques ou immunologiques ni par métabolisme, mais dont la fonction peut être assistée par de tels moyens.

Avantageusement, les compositions selon la description sont adaptées à une administration orale, telles qu'une composition pharmaceutique ou un médicament, un complément alimentaire ou une composition nutraceutique.

Selon une variante, les compositions selon la description sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Les exemples suivants sont donnés pour illustrer l'invention :

Exemple 1: Avocat entier dont noyau 24,5%

3 kg d'avocats d'origine kényane, de calibre 18, de variété *hass* et de dureté mesurée à l'aide d'un pénétromètre, supérieure à 13 Kg/$cm^2$ et dont la part de noyaux représente 24,5% en masse, sont broyés dans un broyeur à couteaux RETSCH du type SM 100 équipé selon la granulométrie souhaitée de tamis de fond à ouverture de maille de 10 mm.

Le broyat est déposé sur un plateau de 0,158 $m^2$, sur une épaisseur de 2 cm.

Le plateau est ensuite disposé dans une étuve ventilée et le séchage est réalisé sous une circulation d'air à 80 ± 5 °C pendant 24 heures.

Le broyat séché est récupéré et broyé pour homogénéisation sur le broyeur à couteaux RETSCH du type SM 100 équipé de tamis de fond à ouverture de maille de 20 mm.

Suivant les essais, l'eau est ensuite ajoutée ou non sur le broyat par vaporisation d'une quantité d'eau purifiée adaptée et le mélange est homogénéisé dans un mélangeur planétaire juste avant d'être introduit dans la presse pour extraction de l'huile.

L'huile d'avocat est ensuite extraite par pression mécanique sur une presse à vis de laboratoire du type Komet préchauffée à la température déterminée de l'essai.

Les quantités d'huile et de tourteau sont déterminées par pesée pour chaque essai et le rendement d'extraction est calculé par la formule suivante :

$$\text{Masse d'huile récupérée x100 / (Masse d'huile récupérée + masse de tourteau).}$$

L'analyse physico-chimique et chromatographique des huiles permet de comparer les compositions des différentes huiles obtenues.

| N° essai | % eau ajoutée après séchage | Température de pression | Rendement d'extraction | Teneur en alkyls polyols |
|---|---|---|---|---|
| | % | °C | % | m/m |
| 1 | 0 | 80 | 15.2 | 2.44 |
| 2 | 3 | 80 | 40.0 | 2.52 |
| 3 | 3 | 100 | 40.4 | 3.01 |

Les essais n°1 et 2 mettent en évidence que l'addition d'eau aux fruits séchés avant pression permet d'atteindre un rendement de pression élevé dans le cas de l'essai n°2, contrairement à l'essai n°1 dans lequel aucune opération d'addition d'eau n'a été mise en œuvre.

Les essais 2 et 3, comparativement à l'essai 1, mettent en évidence le bénéfice cumulé de la température et de l'addition d'eau aux avocats secs sur le rendement de pression et sur la composition de l'huile, notamment sur sa teneur en alkyls polyols.

Exemple 2: Avocat entier dont noyau 25%

3 kg d'avocats d'origine kényane, de calibre 18, de variété *hass* et de dureté mesurée à l'aide d'un pénétromètre, supérieure à 13 Kg/$cm^2$ et dont la part de noyaux représente 25% en masse, sont tranchés en lamelles de 2 à 5 mm, puis répartis en couches régulières sur des plateaux.

Le plateau est ensuite disposé dans une étuve ventilée et le séchage est réalisé sous une circulation d'air à 80 ± 5 °C pendant 24 heures.

Les lamelles séchées est récupérées et broyées pour homogénéisation sur le broyeur à couteaux RETSCH du type SM 100 équipé de tamis de fond à ouverture de maille de 20 mm.

Suivant les essais, l'eau est ensuite ajoutée ou non sur le broyat par vaporisation d'une quantité d'eau purifiée adaptée et le mélange est homogénéisé dans un mélangeur planétaire juste avant d'être introduit dans la presse pour extraction de l'huile.

L'huile d'avocat est ensuite extraite par pression mécanique sur une presse à vis de laboratoire du type Komet

préchauffée à la température déterminée de l'essai.

Les quantités d'huile et de tourteau sont déterminées par pesée pour chaque essai et le rendement d'extraction est calculé par la formule suivante :

Masse d'huile récupérée x100 / (Masse d'huile récupérée + masse de tourteau).

L'analyse physico-chimique et chromatographique des huiles permet de comparer les compositions des différentes huiles obtenues.

| N° essai | % eau ajoutée après séchage | Température de pression | Rendement d'extraction | Teneur en alkyls polyols |
|---|---|---|---|---|
| | % | °C | % | m/m |
| 4 | 0 | 80 | 28,8 | 3,02 |
| 5 | 3 | 100 | 40.8 | 3.19 |

Les essais 4 et 5 mettent en évidence le bénéfice cumulé de la température et de l'addition d'eau aux avocats secs sur le rendement de pression et sur la composition de l'huile, notamment sur sa teneur en alkyls polyols.

Exemple 3 comparatif : pulpe seule

100 kg de pulpe fraîche, mise en purée, sont étalés sur des plateaux en fines couches de quelques millimètres et le séchage est opéré en étuve ventilée à une température de 80°C pendant 24 heures. Le produit séché est ensuite broyé dans un broyeur à marteaux.

29,5 kg de pulpe sèche sont récupérés représentant une perte au séchage de 70,5%.

L'huile est extraite par pression mécanique sur une presse de laboratoire du type Komet à une température de 100°C et après addition d'eau aux avocats séchés par ajout de 2% d'eau et homogénéisation. L'huile est ensuite filtrée sur filtre cloche sous pression d'azote et conditionnée sous atmosphère d'azote.

Les analyses chromatographiques de l'huile ont donné les résultats suivants (% en masse) :

Teneur en 1,2-dihydroxy-4-oxo-aliphatiques alcools et leurs dérivés acétylés de type «persins» et alkyls furanes : 4,63% ;
Teneur en alkyls polyols et leurs dérivés acétylés : 1,60% ;
Teneur en stérols : 0,58% ;
Teneur en hydrocarbures aliphatiques saturés : 0,01%.

Le protocole opératoire décrit dans l'exemple 3 a été reproduit ensuite dans les exemples suivants présentés ci-dessous. Les pourcentages sont exprimés en masse.

Exemple 4 : mélange pulpe + noyau 20%

80 kg de pulpe fraiche, sont broyés avec 20 kg de noyaux, puis étalés sur des plateaux en un film mince de quelques millimètres d'épaisseur.

33,5 kg de produit sec sont récupérés représentant une perte au séchage de 66,5%.

Les analyses chromatographiques de l'huile ont donné les résultats suivants :

Teneur en 1,2-dihydroxy-4-oxo-aliphatiques alcools et leurs dérivés acétylés de type «persins» et alkyls furanes: 4,78% ;
Teneur en alkyls polyols et leurs dérivés acétylés : 1,70% ;
Teneur en stérols : 0,60% ;
Teneur en hydrocarbures aliphatiques saturés : 0,01%.

L'utilisation de 20% de noyau dans le mélange avocat mis en œuvre permet d'augmenter de 6% la teneur en alkyls polyols et leurs dérivés acétylés par rapport à l'huile obtenue à partir de pulpe seule (exemple 3).

Exemple 5 : mélange pulpe + noyau 40%

60 kg de pulpe fraiche, sont broyés avec 40 kg de noyaux, puis étalés sur des plateaux en un film mince de quelques millimètres d'épaisseur.
37,5 kg de produit sec sont récupérés représentant une perte au séchage de 62,5%.
Les analyses chromatographiques de l'huile ont donné les résultats suivants :

Teneur en 1,2-dihydroxy-4-oxo-aliphatiques alcools et leurs dérivés acétylés de type «persins» et alkyls furanes : 5,00% ;
Teneur en alkyls polyols et leurs dérivés acétylés : 1,86% ;
Teneur en stérols : 0,62% ;
Teneur en hydrocarbures aliphatiques saturés : 0,01%.

L'utilisation de 40% de noyau dans le mélange avocat mis en œuvre permet d'augmenter de 16% la teneur en alkyls polyols et leurs dérivés acétylés par rapport à l'huile obtenue à partir de pulpe seule (exemple 3).

Exemple 6 : Avocats durs entiers dont noyau 11%

La force de résistance à la pénétration moyenne des avocats est supérieure à 13 kg/$cm^2$.
100 kg d'avocats entiers frais sont tranchés en lamelles de 2 à 5 mm puis répartis en couches régulières sur des plateaux.
31,2 kg d'avocats séchés sont récupérés représentant une perte au séchage de 68,8 %.
Les analyses chromatographiques de l'huile ont donné les résultats suivants :

Teneur en 1,2-dihydroxy-4-oxo-aliphatiques alcools et leurs dérivés acétylés de type «persins» et alkyls furanes : 4,63% ;
Teneur en alkyls polyols et leurs dérivés acétylés : 1,63% ;
Teneur en stérols : 0,61% ;
Teneur en hydrocarbures aliphatiques saturés : 0,04%.

L'utilisation de 11% de noyau dans le mélange avocat mis en œuvre permet d'augmenter de 2% la teneur en alkyls polyols et leurs dérivés acétylés par rapport à l'huile obtenue à partir de pulpe seule (exemple 3).

Exemple 7 : Avocats durs entiers + noyau (29%)

Le noyau est présente ici à une teneur de 29% en masse dans le mélange de départ mis en œuvre (Avocats durs entiers + noyau).
La force de résistance à la pénétration moyenne des avocats est supérieure à 13 kg/$cm^2$.
80 kg d'avocat durs entiers frais auxquels sont ajoutés 20 kg de noyaux humides sont tranchés en lamelles de 2 à 5 mm puis répartis en couches régulières sur des plateaux.
34,8 kg d'avocats séchés sont récupérés représentant une perte au séchage de 65,2 %.
Les analyses chromatographiques de l'huile ont donné les résultats suivants :

Teneur en 1,2-dihydroxy-4-oxo-aliphatiques alcools et leurs dérivés acétylés de type «persins» et alkyls furanes : 4,81% ;
Teneur en alkyls polyols et leurs dérivés acétylés : 1,76% ;
Teneur en stérols : 0,63% ;
Teneur en hydrocarbures aliphatiques saturés : 0,04%.

L'utilisation de 29% de noyau dans le mélange avocat mis en œuvre permet d'augmenter de 10% la teneur en alkyls polyols et leurs dérivés acétylés par rapport à l'huile obtenue à partir de pulpe seule (exemple 3).

Exemple 8 : Avocats durs entiers + noyau (47%)

Le noyau est présente ici à une teneur de 47% en masse dans le mélange de départ mis en œuvre (fruits durs entiers + noyau).
La force de résistance à la pénétration moyenne des avocats est supérieure à 13 kg/$cm^2$.
60 kg d'avocats entiers frais auxquels sont ajoutés 40 kg de noyaux humides sont tranchés en lamelles de 2

à 5 mm puis répartis en couches régulières sur des plateaux.

38,5 kg d'avocats séchés sont récupérés représentant une perte au séchage de 61,5 %.

Les analyses chromatographiques de l'huile ont donné les résultats suivants :

Teneur en 1,2-dihydroxy-4-oxo-aliphatiques alcools et leurs dérivés acétylés de type «persins» et alkyls furanes : 5,10% ;
Teneur en alkyls polyols et leurs dérivés acétylés : 1,96% ;
Teneur en stérols : 0,66% ;
Teneur en hydrocarbures aliphatiques saturés : 0,04%.

L'utilisation de 47% de noyau dans le mélange avocat mis en œuvre permet d'augmenter de 22,5% la teneur en alkyls polyols et leurs dérivés acétylés par rapport à l'huile obtenue à partir de pulpe seule (exemple 3)

Exemple 9 comparatif : Avocats mous entiers dont noyau (15%)

La force de résistance à la pénétration moyenne des avocats est inférieure à 2 kg/$cm^2$.

100 kg d'avocat mous entiers sont broyés dans un broyeur à couteaux, puis étalés sur des plateaux en un film mince de quelques millimètres d'épaisseur.

35,2 kg d'avocats séchés sont récupérés représentant une perte au séchage de 64,8 %.

Les analyses chromatographiques de l'huile ont donné les résultats suivants :

Teneur en 1,2-dihydroxy-4-oxo-aliphatiques alcools et leurs dérivés acétylés de type «persins» et alkyls furanes : 3,58% ;

Teneur en alkyls polyols et leurs dérivés acétylés : 1,71% ;

Teneur en stérols : 0,66% ;

Teneur en hydrocarbures aliphatiques saturés : 0,03%.

L'utilisation de 15% de noyau dans le mélange avocat mis en œuvre permet d'augmenter de 7% la teneur en alkyls polyols et leurs dérivés acétylés par rapport à l'huile obtenue à partir de pulpe seule (exemple 3).

Le tableau ci-dessous synthétise les résultats des différents essais 3 à 9 :

| N° essai | Matière extraite d'avocat | Teneur en alkyls polyols et dérivés acétylés | Augmentation par rapport à la pulpe seule |
|---|---|---|---|
| | | % m/m | % |
| 3 | Pulpe | 1,60 | - |
| 4 | 80% Pulpe<br>20% noyau | 1,70 | 6 |
| 5 | 60% Pulpe<br>40% noyau | 1,86 | 16 |
| 6 | Avocats durs entiers dont noyau 11% | 1,63 | 2 |
| 7 | Avocats durs entiers dont noyau 29% | 1,76 | 10 |
| 8 | Avocats durs entiers dont noyau 47% | 1,96 | 22,5 |
| 9 | Avocats mous entiers dont noyau 15% | 1,71 | 7 |

## Revendications

1. Procédé d'obtention d'une huile d'avocat enrichie en alkyls polyols et/ou en leurs dérivés acétylés à partir d'au moins des noyaux d'avocats, ladite huile contenant au moins 0,5% en masse d'alkyls polyols et/ou de leurs dérivés acétylés, par rapport à la masse totale de l'huile, lesdits noyaux d'avocat représentant 10 à 50% en masse par rapport à la masse totale d'avocats utilisés, lesdits avocats utilisés n'étant pas des avocats mous ayant une force de résistance à la pénétration dans la chair inférieure ou égale à 3 $kg/cm^2$, ladite force de résistance étant mesurée à l'aide d'un pénétromètre, et ledit procédé comprenant les étapes successives suivantes :

   (1) Tranchage ou broyage d'avocats contenant une teneur en noyaux de 10 à 50% m/m, ledit broyage conduisant à l'obtention d'une granulométrie de broyat comprise entre 2 et 20 mm,
   (2) Séchage à haute température comprise entre 60 et 150°C, jusqu'à l'obtention d'une humidité résiduelle inférieure ou égale à 5%,
   (3) Addition d'eau aux avocats séchés par ajout de 1 à 5 % d'eau ou de vapeur d'eau par rapport à la masse des avocats secs, puis
   (4) Extraction de l'huile par pression mécanique.

2. Procédé d'obtention d'un insaponifiable d'avocat enrichi en alkyls polyols et/ou en leurs dérivés acétylés selon la revendication 1, **caractérisé en ce que** le séchage (2) est réalisé à une température comprise entre 80 et 120°C.

3. Procédé d'obtention d'un insaponifiable d'avocat enrichi en alkyls polyols et/ou en leurs dérivés acétylés selon la revendication 1, **caractérisé en ce que** l'extraction de l'huile (4) par pression mécanique est réalisée à une température comprise entre 80 et 100°C.

4. Procédé d'obtention d'une huile d'avocat enrichie en alkyls polyols et/ou en leurs dérivés acétylés selon la revendication 1, **caractérisé en ce que** les noyaux d'avocat représentent 15 à 40% en masse, par rapport à la masse totale d'avocats utilisés.

5. Procédé d'obtention d'une huile d'avocat enrichie en alkyls polyols et/ou en leurs dérivés acétylés selon la revendication 4, **caractérisé en ce que** les noyaux d'avocat représentent 20 à 40% en masse, par rapport à la masse totale d'avocats utilisés.

6. Procédé d'obtention d'une huile d'avocat enrichie en alkyls polyols et/ou en leurs dérivés acétylés selon l'une quelconque des revendications 1 à 5, **caractérisé en ce que** les alkyls polyols sont des triols du type 1,2,4-trihydroxy à chaînes aliphatiques non ramifiées en C17 à C21, saturées, mono ou polyinsaturées.

7. Procédé d'obtention d'une huile d'avocat enrichie en alkyls polyols et/ou en leurs dérivés acétylés selon la revendication 6, caractérisé en que les alkyls polyols sont choisis dans le groupe constitué par le nonadecane-1,2,4-triol; le heneicosa-cis, cis-12-15 diene-1,2,4-triol; le heptadec-16-yne-1,2,4-triol; le heptadec-cis-16-ene-1,2,4-triol ; et leurs mélanges.

8. Huile d'avocat enrichie en alkyls polyols et/ou en leurs dérivés acétylés, susceptible d'être obtenue par le procédé selon l'une quelconque des revendications 1 à 7, **caractérisée en ce qu'**elle contient au moins 0,5% en masse d'alkyls polyols ou de leurs dérivés acétylés, par rapport à la masse totale de l'huile.

9. Huile d'avocat enrichie en alkyls polyols et/ou en leurs dérivés acétylés, selon la revendication 8, **caractérisée en ce qu'**elle contient entre 0,5 et 10% en masse d'alkyls polyols et/ou de leurs dérivés acétylés, par rapport à la masse totale de l'huile.

10. Huile d'avocat enrichie en alkyls polyols et/ou en leurs dérivés acétylés selon la revendication 8 ou 9, **caractérisée en ce qu'**elle contient des stérols et/ou des hydrocarbures aliphatiques saturés.

11. Huile d'avocat enrichie en alkyls polyols et/ou en leurs dérivés acétylés selon l'une quelconques des revendications 8 à 10, **caractérisée en ce qu'**elle contient des 1,2-dihydroxy-4-oxo-aliphatiques alcools et leurs dérivés acétylés de type «persins» et/ou des alkyls furanes.

12. Insaponifiable d'avocat enrichi en alkyls polyols ou concentrat d'huile d'avocat enrichi en alkyls polyols et/ou en leurs dérivés acétylés, ledit concentrat contenant au moins 5% d'alkyls polyols et/ou de leur dérivés acétylés en

masse, par rapport à la masse totale du concentrat, ledit insaponifiable et ledit concentrat d'huile étant susceptibles d'être obtenus à partir de l'huile d'avocat telle que définie à l'une quelconque des revendications 8 à 11, pour son utilisation comme médicament.

**13.** Insaponifiable d'avocat enrichi en alkyls polyols ou concentrat d'huile d'avocat enrichi en alkyls polyols et/ou en leurs dérivés acétylés selon la revendication 12, pour son utilisation dans la prévention et/ou le traitement des troubles du tissu conjonctif tels que l'arthrose, des pathologies articulaires telles que les rhumatismes, ou encore des maladies parodontales, telles que la gingivite ou la parodontite.

## Patentansprüche

**1.** Verfahren zur Herstellung eines mit Alkylpolyolen und/oder mit deren acetylierten Derivaten angereicherten Avocadoöls aus mindestens Avocadokernen, wobei das Öl mindestens 0,5 Ma% Alkylpolyole und/oder deren acetylierte Derivate in Bezug auf die Gesamtmasse des Öls enthält, wobei die Avocadokerne 10 bis 50 Ma% in Bezug auf die Gesamtmasse verwendeter Avocados darstellt, wobei die verwendeten Avocados keine weichen Avocados sind mit einer Widerstandskraft gegenüber Eindringen in das Fleisch von unter oder gleich 3 $kg/cm^2$, wobei die Widerstandskraft mit Hilfe eines Penetrometers gemessen wird, und wobei das Verfahren die folgenden aufeinanderfolgenden Schritte umfasst:

(1) Schneiden oder Zerkleinern von Avocados mit einem Gehalt an Kernen von 10 bis 50 % m/m, wobei das Zerkleinern zu einer Mahlgut-Teilchengröße zwischen 2 und 20 mm führt,
(2) Trocknen bei hoher Temperatur zwischen 60 und 150 °C bis zum Erhalt einer Restfeuchte von unter oder gleich 5 %,
(3) Hinzufügen von Wasser zu den getrockneten Avocados durch Hinzugabe von 1 bis 5 % Wasser oder Wasserdampf in Bezug auf die Masse der trockenen Avocados, dann
(4) Extrahieren des Öls durch mechanisches Pressen.

**2.** Verfahren zur Herstellung eines mit Alkylpolyolen und/oder mit deren acetylierten Derivaten angereicherten Avocado-Unverseifbaren nach Anspruch 1, **dadurch gekennzeichnet, dass** das Trocknen (2) bei einer Temperatur zwischen 80 und 120 °C durchgeführt wird.

**3.** Verfahren zur Herstellung eines mit Alkylpolyolen und/oder mit deren acetylierten Derivaten angereicherten Avocado-Unverseifbaren nach Anspruch 1, **dadurch gekennzeichnet, dass** die Extraktion des Öls (4) durch mechanisches Pressen bei einer Temperatur zwischen 80 und 100 °C durchgeführt wird.

**4.** Verfahren zur Herstellung eines mit Alkylpolyolen und/oder mit deren acetylierten Derivaten angereicherten Avocadoöls nach Anspruch 1, **dadurch gekennzeichnet, dass** die Avocadkerne 15 bis 40 Ma% in Bezug auf die Gesamtmasse verwendeter Avocados darstellen.

**5.** Verfahren zur Herstellung eines mit Alkylpolyolen und/oder mit deren acetylierten Derivaten angereicherten Avocadoöls nach Anspruch 4, **dadurch gekennzeichnet, dass** die Avocadkerne 20 bis 40 Ma% in Bezug auf die Gesamtmasse verwendeter Avocados darstellen.

**6.** Verfahren zur Herstellung eines mit Alkylpolyolen und/oder mit deren acetylierten Derivaten angereicherten Avocadoöls nach einem der Ansprüche 1 bis 5, **dadurch gekennzeichnet, dass** die Alkylpolyole Triole vom Typ 1,2,4-Trihydroxy mit nicht verzweigten aliphatischen Ketten in C17 bis C21, gesättigt, mono- oder mehrfach ungesättigt, sind.

**7.** Verfahren zur Herstellung eines mit Alkylpolyolen und/oder mit deren acetylierten Derivaten angereicherten Avocadoöls nach Anspruch 6, **dadurch gekennzeichnet, dass** die Alkylpolyole aus der Gruppe ausgewählt sind, die von dem Nonadecan-1,2,4-triol; dem Heneicosa-cis, cis-12-15 dien-1,2,4-triol; dem Heptadec-16-yn-1,2,4-triol; dem Heptadec-cis-16-en-1,2,4-triol und deren Gemischen gebildet ist.

**8.** Avocadoöl, angereichert mit Alkylpolyolen und/oder mit deren acetylierten Derivaten, herstellbar durch das Verfahren nach einem der Ansprüche 1 bis 7, **dadurch gekennzeichnet, dass** es mindestens 0,5 Ma% Alkylpolyole oder deren acetylierte Derivate in Bezug auf die Gesamtmasse des Öls enthält.

9. Avocadoöl, angereichert mit Alkylpolyolen und/oder mit deren acetylierten Derivaten nach Anspruch 8, **dadurch gekennzeichnet, dass** es zwischen 0,5 und 10 Ma% Alkylpolyole oder deren acetylierte Derivate in Bezug auf die Gesamtmasse des Öls enthält.

10. Avocadoöl, angereichert mit Alkylpolyolen und/oder mit deren acetylierten Derivaten nach Anspruch 8 oder 9, **dadurch gekennzeichnet, dass** es Sterole und/oder gesättigte aliphatische Kohlenwasserstoffe enthält.

11. Avocadoöl, angereichert mit Alkylpolyolen und/oder mit deren acetylierten Derivaten nach einem der Ansprüche 8 bis 10, **dadurch gekennzeichnet, dass** es 1,2-dihydroxy-4-oxo-aliphatische Alkohole und deren acetylierte Derivate vom Typ "Persin" und/oder Alkylfurane enthält. enthält.

12. Avocado-Unverseifbares, angereichert mit Alkylpolyolen und/oder mit deren acetylierten Derivaten, wobei das Konzentrat mindestens 5 Ma% Alkylpolyole und/oder deren acetylierte Derivate in Bezug auf die Gesamtmasse des Konzentrats enthält, wobei das Unverseifbare und das Ölkonzentrat aus Avocadoöl nach einem der Ansprüche 8 bis 11 herstellbar sind für dessen Verwendung als Arzneimittel.

13. Avocado-Unverseifbares, angereichert mit Alkylpolyolen oder Avocadoölkonzentrat, angereichert mit Alkylpolyolen und/oder mit deren Derivaten nach Anspruch 12 für dessen Verwendung zur Vorbeugung und/oder Behandlung von Erkrankungen des Bindegewebes wie der Arthrose, der Gelenkerkrankungen wie Rheuma oder auch von Parodontalkrankheiten wie der Gingivitis oder der Parodontitis.

## Claims

1. A method for obtaining avocado oil enriched in alkyl polyols and/or acetylated derivatives thereof from at least avocado seeds, said oil containing at least 0.5% alkyl polyols and/or acetylated derivatives thereof by weight, relative to the total weight of oil, said avocado seeds representing 10 to 50% by weight relative to the total weight of avocados used, said avocados used not being soft avocados having a pulp penetration resistance of less than or equal to 3 $kg/cm^2$, said resistance being measured using a penetrometer, and said method comprising the following successive steps:

   (1) Cutting or grinding of avocados with a seed content of 10 to 50% w/w, said grinding producing a particle size distribution of the ground material between 2 and 20 mm,
   (2) High-temperature drying at a temperature between 60 and 150 °C, until a residual moisture content less than or equal to 5% is obtained,
   (3) Addition of water to the dried avocados by adding 1 to 5% water or water vapor relative to the weight of dry avocados, then
   (4) Oil extraction by mechanical pressure.

2. The method for obtaining avocado unsaponifiable enriched in alkyl polyols and/or acetylated derivatives thereof according to claim 1, **characterized in that** drying (2) is carried out at a temperature of between 80 and 120°C.

3. The method for obtaining avocado unsaponifiable enriched in alkyl polyols and/or acetylated derivatives thereof according to claim 1, **characterized in that** oil extraction (4) by mechanical pressure is carried out at a temperature between 80 and 100 °C.

4. The method for obtaining avocado oil enriched in alkyl polyols and/or acetylated derivatives thereof according to claim 1, **characterized in that** avocado seeds represent 15 to 40% by weight, relative to the total weight of avocados used.

5. The method for obtaining avocado oil enriched in alkyl polyols and/or acetylated derivatives thereof according to claim 4, **characterized in that** avocado seeds represent 20 to 40% by weight, relative to the total weight of avocados used.

6. The method for obtaining avocado oil enriched in alkyl polyols and/or acetylated derivatives thereof according to any one of claims 1 to 5, **characterized in that** the alkyl polyols are saturated, monounsaturated or polyunsaturated triols of the $C_{17}$ to $C_{21}$ aliphatic unbranched linear 1,2,4-trihydroxy type.

7. The method for obtaining avocado oil enriched in alkyl polyols and/or acetylated derivatives thereof according to claim 6, **characterized in that** the alkyl polyols are selected from the group comprised of nonadecane-1,2,4-triol; heneicosa-cis, cis-12-15 diene-1,2,4-triol; heptadec-16-yne-1,2,4-triol; heptadec-cis-16-ene-1,2,4-triol; and mixtures thereof.

8. Avocado oil enriched in alkyl polyols and/or acetylated derivatives thereof, obtainable by the method according to any one of claims 1 to 7, **characterized in that** it contains at least 0.5% alkyl polyols or acetylated derivatives thereof by weight, relative to the total weight of oil.

9. The avocado oil enriched in alkyl polyols and/or acetylated derivatives thereof according to claim 8, **characterized in that** it contains between 0.5 and 10% alkyl polyols and/or acetylated derivatives thereof by weight, relative to the total weight of oil.

10. The avocado oil enriched in alkyl polyols and/or acetylated derivatives thereof according to claim 8 or 9, **characterized in that** it contains sterols and/or saturated aliphatic hydrocarbons.

11. The avocado oil enriched in alkyl polyols and/or acetylated derivatives thereof according to any one of claims 8 to 10, **characterized in that** it contains 1,2-dihydroxy-4-oxo-aliphatic alcohols and acetylated derivatives thereof of the "persins" type and/or alkyl furans.

12. Avocado unsaponifiable enriched in alkyl polyols or concentrate of avocado oil enriched in alkyl polyols and/or acetylated derivatives thereof, said concentrate containing at least 5% alkyl polyols and/or acetylated derivatives thereof by weight, relative to the total weight of the concentrate, said unsaponifiable and said oil concentrate being obtainable from avocado oil as defined in any one of claims 8 to 11, for use as a drug.

13. The avocado unsaponifiable enriched in alkyl polyols or concentrate of avocado oil enriched in alkyl polyols and/or acetylated derivatives thereof according to claim 12, for use in the prevention and/or treatment of conjunctive tissue disorders such as arthrosis, articular pathologies such as rheumatism, or periodontal diseases such as gingivitis or periodontitis.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013098393 A


### Littérature non-brevet citée dans la description


- Extraction, characterization, and prediction of the oxidative stability of avocado oil. **BIZIMANA V.** Dissertation. University of Minnesota, 1997, 1-241